# EUROPÄISCHE PATENTSCHRIFT

(11) **EP 0 782 591 B1**
(45) Veröffentlichungstag und Bekanntmachung des Hinweises auf die Patenterteilung: **07.01.1999**
(21) Anmeldenummer: 95929770.6
(22) Anmeldetag: 06.09.1995
(51) Int. Cl.: C08F 297/02, A61K 6/083

(54) **POLYMERISIERBARE OLIGO- UND/ODER POLYALKENSÄUREN**
POLYMERISABLE OLIGO AND/OR POLYALKENE ACIDS
OLIGO-ACIDES ET/OU ACIDES DE POLYALCENE POLYMERISABLES

(30) Priorität: 23.09.1994 DE 4433987
(43) Veröffentlichungstag der Anmeldung: 09.07.1997
(73) Patentinhaber: ERNST MÜHLBAUER KG, D-22547 Hamburg (DE)
(72) Erfinder: THOMSEN, Sven, Arne, D-69221 Dossenheim (DE)
(74) Vertreter: Schupfner, Gerhard D.
(86) Internationale Anmeldenummer: DE9501218
(87) Internationale Veröffentlichungsnummer: WO9609332

(56) Entgegenhaltungen:
- EP-A- 0 024 056
- EP-A- 0 219 058
- EP-A- 0 298 667
- EP-A- 0 430 705
- DE-A- 4 229 947

## Beschreibung

Die Biomaterialforschung hat in den letzten Jahren auf dem Gebiet der synthetischen dentalen und medizinischen Restaurationsmaterialien eine Reihe neuer und optimierter Werkstoffe hervorgebracht. Der Wunsch nach Materialien, die bezüglich ästhetisch-kosmetischer Kriterien der natürlichen Zahnsubstanz möglichst nahe kommen, und trotzdem eine einwandfreie medizinische Versorgung gewährleisten, steht hier im Vordergrund. Folglich begann man schon sehr früh, das häufig verwendete Amalgam aufgrund seiner Eigenfarbe im Frontzahnbereich gegen Zahnzemente einzutauschen.

Klassische Dentalzemente bestehen in der Regel aus feingemahlenen Metalloxiden, Metallhydroxiden oder anderen ionenabgebenden Materialien wie Gläsern und einer wäßrigen Säure. Die bekanntesten Zemente dieses Typs sind der Phosphatzement (Zinkoxid + Phosphorsäure) und der Silikatzement (Glaspulver + Phosphorsäure). Sie sind demnach rein anorganischer Struktur, haften nur wenig an der Zahnsubstanz und sind relativ spröde. Aus diesem Grund, und weil sie stark pulpareizend wirken, sind sie fast völlig vom Markt verschwunden.

Nach der Entdeckung der Polyacrylsäure wurden etwa ab Mitte dieses Jahrhunderts zunehmend modifizierte organische oder halborganische Zemente eingeführt. Sie bestehen wiederum aus einem Metalloxid oder -hydroxid und einer relativ hochmolekularen organischen Säurekomponente. In diesem Zusammenhang sind vor allem die Carboxylatzemente (Zinkoxid + Polyacrylsäure) zu nennen. Sie werden als Wurzelfüllungsmaterial oder zur Überkappung der Pulpa verwendet. Durch ihren hohen pH-Wert wirken sie als schützende Sperre gegen Säuren und andere toxische Substanzen, die in einigen Füllungsmaterialien enthalten sein können. Das Hauptproblem dieser Zemente ist jedoch, daß die Abbindereaktion sehr langsam verläuft, die mechanische Belastbarkeit gering ist und die Materialien durch wäßrige Medien ausgewaschen werden können.

Die großen Nachteile der Dentalzemente haben dazu geführt, daß diese weitgehend durch die dauerhafteren, höher zu belastenden, kantenfesteren und unlöslichen Composite ersetzt worden sind. Composite bestehen im wesentlichen aus einem polymerisierbaren Bindemittel, welches durch einen organischen oder anorganischen Füllstoff verstärkt ist. Als polymerisierbare Bindemittel eignen sich vor allem Verbindungen mit olefinisch ungesättigten Gruppen, vorzugsweise Ester der (Meth)acrylsäure von ein- und mehrwertigen Alkoholen. Als anorganische Füllstoffe verwendet man feine Quarzmehle, mikrofeine Kieselsäuren, Aluminiumoxid, Bariumgläser oder andere mineralische Teilchen, die zur besseren Haftung mit einem polymerisierbaren Silan umgeben werden. Wesentlich für Composite ist, daß ihre Aushärtung durch eine radikalische Polymerisationsreaktion der olefinischen Doppelbindungen abläuft und nicht der Gegenwart von Wasser bedarf. Obwohl heute neben Amalgamen hauptsächlich Composites als dentales Restaurationsmaterial eingesetzt werden, sind auch ihnen in der Anwendung Grenzen gesetzt. Gewebeirritationen und Toxizitäten bei tiefergehenden Zahnkavitäten, sowie Polymerisationsschrumpf und fehlende Haftung am Zahn schränken ihre Anwendung ein.

Ein großer Durchbruch in der Dentalforchung gelang Ende der sechziger Jahre mit der Entdeckung der Glas-Ionomer-, oder besser Glas-Polyalkenoat-Zemente. Diese Glas-Polyalkenoat-Zemente sind ebenfalls Mischungen aus organischen Komponenten (z.B.: Polyalkensäuren, Hydroxycarbonsäuren) und anorganischen Komponenten (z.B.: Aluminiumsilikatgläsern, Quarzen), wobei als Reaktionsmedium für die Zementreaktion Wasser benötigt wird. Die Zementreaktion ist, wie bei anderen Zementen auch, eine Säure-Base-Reaktion, bei der die Polycarbonsäure als Protonendonator und das Alumosilikatglas als Protonenakzeptor fungiert. Die Reaktion läßt sich formal in drei Schritte unterteilen, wobei jedoch die Grenzen fließend sind:
1. Hydrolyse, oder besser Dissoziation, der Säuregruppen und Herauslösen von Kationen (zuerst Ca²⁺, später Al³⁺) aus dem Glas.
2. Salzbildung zwischen Carboxylatgruppen und Kationen, wobei Gelbildung und/oder Ausfällung des Salzes eintritt.
3. Langsames Ausbilden von Kationenbrücken, welche die Polycarbonsäure vernetzen und die mechanischen Eigenschaften verbessern. Der Zement wird unlöslich.

Die Glas-Polyalkenoat-Zemente sind seit 1971 in der Literatur beschrieben ( A. D. Wilson, J. Appl. Chem. Biotechnol. 21,313(1971)) und werden seither als Füllungs- und Unterfüllungsmaterialien, sowie als Klebezemente in der Zahnheilkunde verwendet. Gegenüber den klassischen Zementen und Compositen haben sie entscheidende Vorteile wie Bioverträglichkeit, Beständigkeit im Mund, gute Haftung am Zahn und gutes Aussehen bzw. Opazität.

In den letzten 20 Jahren seit ihrer Erfindung setzten intensive Bemühungen ein, die schon recht guten Eigenschaften weiter zu verbessern und neue Anwendungsgebiete zu erschließen. So wurden beispielsweise Gläser verwendet, die Fluoridionen an das benachbarte Zahnmaterial abgeben, oder radioopake Stoffe zugemischt, um die Röntgensichtbarkeit zu erhöhen. Doch auch diese Glas-Polyalkenoat-Zemente erfüllten nicht alle Erwartungen. Sie sind insbesondere gegenüber Feuchtigkeit während der frühen Aushärtphase zu empfindlich.

Die Lösung dieses Problems sind die polymerisierbaren Glas-Polyalkenoat-Zemente. Sie stellen eine sehr interessante neue Materialklasse dar, weil sie die Vorteile der Composites (hohe mechanische Belastbarkeit, Unlöslichkeit) mit denen der Glas-Polyalkenoat-Zemente (gute Haftung am Zahn, Fluoridionenfreisetzung) verbinden. Polymerisierbare Glas-Polyalkenoat-Zemente bestehen aus einem ionenfreisetzenden Alumosilikatpulver und einer jetzt polymerisierbaren Säurekomponente. Diese polymerisierbaren Säuren sind meist hydrophile ungesättigte Carboxyl- oder Phosphorsäureverbindungen, wie z.B. oligomere Homo- oder Copolymerisate der (Meth)acrysäure und/oder ihrer Derivate. Sie fungieren nach dem Anmischen als Bindeglied zwischen der wasserhaltigen Glasionomermatrix und der hydrophoben Kunststoffmatrix.

Die Anwendung polymerisierbarer Säuren beschränkt sich jedoch nicht nur auf polymerisierbare Glasionomerzemente. Ebenso gute Eigenschaften haben sie in dentalen Haftvermittlern, sogenannte Bonds und oberflächenmodifizierenden Präparaten sogenannte Preps oder Conditioner. Einen Überblick über die große Zahl von polymerisierbaren Säuren und ihren Derivaten wird in EP-B1 0 219 058 der Ernst Mühlbauer KG gegeben. Die bisher eingesetzten Verbindungen können im wesentlichen in zwei Gruppen unterteilt werden:
1. Kurzkettige Verbindungen, wie modifizierte Vinylmonomere (siehe auch DE-A1 41 41 174) oder niedermolekulare Acryl-, bzw. Methacrylsäurederivate, z.B. N-Tolylglycin-N-glycerinmethacrylat oder Pyromellitsäuredimethacrylat "PMDM" (siehe auch EP-A2 0 391 619). In diese Gruppe fallen auch 4-(2-Methacryloyloxyethyl)trimellitsäureanhydrid, "4-META" (EP-A1 0 425 200), Dipentaerythrit-pentaacrylat-phosphorsäureester "PENTA" (EP-Al 0 554 890) oder das sogenannte TCB-Harz , welches ein Butantetracarbonsäure-bis-HEMA-ester ist (HEMA=Hydroxyethyl-methacrylat) (EP-A2 0 499 180).
2. Langkettige Verbindungen, wie ungesättigte Polycarbonsäuren. Bevorzugt werden Copolymerisate aus Acryl- und Maleinsäure oder Glycidyl-Polymethacrylat-Addukte (s. auch EP-A2 0 329 268).

DE 42 29 947 offenbart Dentalzemente, deren Polymerkomponenete aus folgenden Monomerbausteinen besteht: Vinyldicarbonsäureanhydriden bzw. deren Umsetzungsprodukte mit hydroxyfunktionellen Verbindungen und/oder anderen Vinylpolymeren als Copolymeren. Die Seitenketten/-gruppen der Polymere weisen keine Doppelbindung auf. Die Polymere werden durch radikalische Polymerisation erhalten und damit sind die Monomere in dem Polymerisat statistisch verteilt.

EP-A-0 298 667 offenbart Methacrylsäure-Blockpolymerisate, enthält jedoch keinen Hinweis auf Blockpolymerisate, die ungesättigte Ester/Amide mit zusätzlicher Doppelbindung in der Alkohol-/ Aminkomponente als Monomerbaustein enthalten.

Nachteilig bei der Verwendung von einer der beiden ungesättigten Verbindungsklassen ist jedoch immer noch die vergleichsweise niedrige Endfestigkeit des zementes im Vergleich zu den Composites. Es wird hiermit deutlich, daß derzeit noch kein idealer, bzw. perfekter Dentalzement verfügbar ist.

Aufgabe der Erfindung ist es, neue polymerisierbare Säuren mit definierter und nach Wunsch auf die jeweiligen Erfordernisse einstellbarer Struktur herzustellen. Hierzu soll ein reproduzierbares und möglichst universell einsetzbares Syntheseverfahren entwickelt werden.

Diese Aufgabe wird erfindungsgemäß durch die Bereitstellung einer neuen Klasse von Blockpolymeren aus polymerisierbaren Oligo- und/oder Polyalkensäuren der allgemeinen Formel

A-[-Bₓ-C_{y}-D_{z}-]ₙ-E

gelöst, wobei die Gruppen **A** und **E** gleich oder verschieden Wasserstoff, Halogenatome, Methacrylat-, Acrylat-, Allyl-, Vinyl-, Alkyl-, Aryl-, Alkoxy-, Aryloxy-, Carboxyl-, Amin, Hydroxy-, Isocyanat-, Silyl- und/oder Siloxygruppen sind und als Endgruppen fungieren.

Das Oligomer/Polymer besteht aus mindestens 2 Blöcken (hier = Gruppen) von Sequenzen, wobei die eine Gruppe B ein Segment bestehend aus Sequenzen von Mono-, Di- und/oder Tricarbonsäuren als Monomerbausteine, deren Anhydriden, Salzen und/oder Derivaten dieser Säuren mit einer an sich bekannten Säureschutzgruppe ist, aus denen die Säure leicht freizusetzen ist. Die andere Gruppe C besteht aus Sequenzen von Mono-, Di- und /oder Tricarbonsäureestern und/oder deren Amiden, wobei die Alkoholkomponente der Ester, sowie die Aminkomponente der Amide jeweils ungesättig ist.

Eine dritte optionale Gruppe D besteht aus Sequenzen von Mono- Di- und /oder Tricarbonsäuren als Monomerbausteine, deren Estern, Amiden und/oder Nitrilen, die keine Doppelbindungen enthalten.

Die Abfolge der Gruppen B ,C und D innerhalb der Blöcke [-Bₓ-C_{y}-D_{z}-] ist beliebig und kann für jedes n unterschiedlich sein.

Die in den Gruppen B, C und D in sequentieller Abfolge enthaltenen Monomerbausteine sind für ein bestimmtes n jeweils gleich können, aber für unterschiedliche n unterschiedlich sein.

Die Indizes bedeuten n= 1 bis 10 und x ,y , z = 0 oder 4 bis 1000 , wobei für mindestens ein n x mindestens 4 und y mindestens 4 sind und x ,y ,z für jedes n unterschiedlich sein können. Bevorzugt ist n=1 bis 4 , x und y mindestens 4 und z=0 oder mindestens auch 4.

In einer erfindungsgemäßen Ausführungsform enthalten die Gruppen B, C und D als Monomerbaustein, der die Oligomer-/Polymerkette bildet, die Säuren Crotonsäure, iso-Crotonsäure, Fumarsäure, Mesaconsäure, 3-Buten-1,2,3-Tricarbonsäure sowie bevorzugt Acrylsäure, Methacrylsäure, Maleinsäure und/oder Itaconsäure.

Die Oligo/Polyalkensäuren der Gruppen B, C und D unterscheiden sich in ihrer Derivatisierung bzw. in der Funktionalität ihrer Reste.

In der Gruppe B liegt die Säure als Säure, als Säureanhydrid, als Salz der Säure oder als eine mit einer Säureschutzgruppe versehene Säure vor, wobei die Säureschutzgruppe bevorzugt ein Trialkylsilylester oder ein Siloxyester und besonders bevorzugt Trimethylsilyl ist

In der Gruppe C ist die Alkoholkomponente des Esters oder Aminkomponente des Amides ein ungesättigter Alkylrest, bevorzugt Vinyl, Allyl und/oder Vinyliden, wenn der Monomerbaustein eine Säure ist.

In der Gruppe D ist die Alkoholkomponente des Esters oder Aminkomponente des Amides ein gesättigter Alkylrest, bevorzugt Methyl, Ethyl, Propyl, tert.-Butyl, und/oder ein substituierter oder unsubstituierter Arylrest.

In einer besonders bevorzugten Ausführungsform der Erfindung ist die Gruppe C ein Segment bestehend aus oligomeren/polymeren Sequenzen des Methacrylsäureallyl- oder vinylester und die Gruppe B ein Segment bestehend aus oligomeren/polymeren Sequenzen des Methacrylsäuretrimethylsilylester.

Verwendung finden diese polymerisierbaren Oligo/Polyalkensäuren im dentalen und medizinischen Bereich, insbesondere als Komponente in Glasionomerzementen. Diese polymerisierbaren Glas-Polyalkenoat-Zemente bestehen aus einem ionenfreisetzenden Alumosilikatpulver. Die Säure fungiert nach dem Anmischen und der möglichen Vernetzung der olefinischen Seitengruppen als Bindeglied zwischen der wasserhaltigen Glasionomermatrix und der hydrophoben Kunststoffmatrix.

Die Anwendung dieser polymerisierbaren Säuren beschränkt sich jedoch nicht nur auf polymerisierbare Glasionomerzemente. Ebenso gute Eigenschaften haben sie in dentalen Haftvermittlern (sogenannten Bonds) und oberflächenmodifizierenden Präparaten (sogenannten Preps oder Conditioner).

Neu ist außerdem, daß diese polymerisierbaren Säuren oder ihre Vorläufer durch anionische Polymerisation als "living polymers" hergestellt werden, und daß erstmals Blockpolymere hergestellt und verwendet werden. Die einzelnen Blöcke sollen sich bei ausreichender Länge wie die reine Substanz verhalten. Durch die Blockstruktur befinden sich die für die Zementreaktion wichtigen Carboxylgruppen und die für die radikalische Vernetzung wichtigen Allylgruppen in räumlicher Nähe, so daß die ansonsten eine Umsetzung behindernde sterische Hinderung bzw. Diffussions-Hinderung der schwer beweglichen Kettensegmente vorteilhaft überwunden wird. Dies ist besonders wichtig, wenn die Zementrektion oder die radikalische Vernetzung mit hochviskosen oder gar als Feststoff vorliegenden Polymeren durchgeführt wird.

Nur durch dieses Verfahren lassen sich polymerisierbare Säuren der obigen Struktur synthetisieren und Blöcke gewünschter Art und Länge herstellen, sowie die beschriebenen Endgruppen einfügen.

Vorzüge der erfindungsgemäßen polymerisierbaren Oligo-/Polyalkensäuren sind eine schon durch die Synthese durch Wahl geeigneter Blocklängen und Abfolgen genau vorgegebene und optimal abstimmbare Anzahl möglicher Säurefunktionen für die Zement reaktion und radikalisch-vernetzungsfähiger olefinischer Seitengruppen. Aushärtevorgang, Endhärte und ästhetisch-kosmetischer Kriterien wie Opazität , des Produktes lassen sich so innerhalb weiter Grenzen variieren und optimieren.

Die Ausführung der anionischen Polymerisation bei tiefen Temperaturen ist vorteilhaft, weil sich so beispielsweise selektiv (Meth)acrylgruppen in Gegenwart von Allylgruppen polymerisieren lassen. Die allylische Doppelbindung bleibt unberührt und steht damit für eine spätere radikalische Vernetzungsreaktion der Polymerstränge zur Verfügung.

Eine radikalische Polymerisation der Monomere würde zur Vernetzung und damit zur Unlöslichkeit der Produkte führen, weil dann (Meth)acrylgruppen und Allylgruppen Polymerreaktionen eingehen. Ein weiteres Aushärten des Zahnzements durch radikalische Vernetzung ist dann nicht mehr möglich.

Bei der anionischen Polymerisation können als Lösungsmittel verwendet werden
a) aliphatische und/oder aromatische Kohlenwasserstoffe wie Toluol, Benzol, Alkane oder Cycloalkane,
b) offenkettige oder cyclische Ether wie Tetrahydrofuran, Dioxan oder Diethylether,
c) Ketone, Heterocyclen (NMP) oder flüssiges Ammoniak.

Als Startersysteme für die anionische Polymerisation können verwendet werden
a) metallorganische Verbindungen, wie prim-, sek-, tert- Butyllithium, Naphthalin-, Biphenyl-, Phenanthren-, oder Anthracenkomplexe der Alkalimetalle Li, K, Na, Grignardverbindungen, sowie Zieglerkatalysatoren oder Kaliumamid, und
b) Alkalimetalle allein.

Die für die anionische Polymerisation verwendeten Monomere und Lösungsmittel müssen rigoros getrocknet und frei von kettenabbrechenden Verunreinigungen sein. Zweckmäßiger Weise werden die Monomere hierfür einen Tag über CaH₂ gerührt und dann unter Stickstoff im Vakuum über eine Kolonne destilliert. Reicht das nicht, werden die Verbindungen über Dialkylmagnesium- oder stabilisierten Grignardverbindungen erneut destilliert. Man lagert über Molsieb.

### Beispiele

### Allgemeine Herstellungsvorschriften

Vor der Polymerisation muß der Reaktor ausgeheizt und mit wasserfreiem Stickstoff gefüllt sein. Nach Abkühlen des Reaktors auf Raumtemperatur wird das Lösungsmittel unter Stickstoff eingefüllt und auf -70 bis -80°C abgekühlt. Man gibt die berechnete Menge des Initiators zu und läßt ebenfalls abkühlen. In diese Mischung wird nun unter Rühren das zu polymerisierende Monomer oder Monomerengemisch auf einmal zugegeben. Die Lösung entfärbt sich, während die Viskosität langsam ansteigt. Nach 30 bis 60 min. kann weiteres Monomer oder Monomerengemisch zudosiert werden. Die Reaktion wird nach weiteren 30 bis 60 min. mit Ethanol abgebrochen.

Die Aufarbeitung richtet sich nach den verwendeten Lösungsmitteln und Monomeren und ist bei jedem Versuch verschieden.

### Beispiel 1

40 ml Tetrahydrofuran werden auf -70 bis -80°C abgekühlt und 1 ml einer 1M Lösung von Naphthalinnatrium in THF (lmmol) zugegeben. Zu der tiefgrünen Lösung gibt man schnell 10 ml (9,38g / 74,4 mmol) Allylmethacrylat und rührt für 30 min. Die Lösung behält eine leichte Gelbfärbung. Anschließend werden 5 ml (4,45g / 28,1 mmol) Methacrylsäuretrimethylsilylester zugegeben und für weitere 30 min. gerührt. Die Reaktion wird mit 2 ml Ethanol abgebrochen und die Trimethylsilylgruppen durch Rühren über Nacht mit 5 ml 10%iger wäßriger HCl-Lösung entfernt. Das Polymer wird ausgefällt, gewaschen und im Vakuum getrocknet. Eine Molekulargewichtsbestimmung mittels GPC ergab ein Molekulargewicht von 35-40000 g/mol.

| IR-Spektrum (cm⁻¹) : | | | |
|---|---|---|---|
| 3500-2500 | COOH | 3090 | =C-H |
| 3000-2900 | -C-H | 1740-1700 | C=O |
| 1650 | C=C | 980 + 920 | Allylgruppe |

Löslichkeitsversuche in Aceton oder Toluol zeigten, daß das Polymer nicht vernetzt ist. Die pulverförmige Substanz wurde in ein polymerisierbares Harz Bis-GMA/HEMA (2.2-Bis-4(2-hydroxy-3-methacryloxy-propyloxy)-phenyl-propan / 2-Hydroxyethyl-methacrylat) eingearbeitet und das Gemisch ausgehärtet. Die mechanischen Werte der Harzmatrix verschlechterten sich durch das Polymer nicht. Auch nach längerer Wasserlagerung blieben die Werte konstant.

### Beispiel 2

20 ml Tetrahydrofuran werden auf -70 bis -80°C abgekühlt und 0,5 ml einer 2M Lösung von n-Butyllithium in Cyclohexan (lmmol) zugegeben. Zu dieser Lösung gibt man schnell 15 ml (13,35 g / 84,5 mmol) Methacrylsäuretrimethylsilylester, worauf die Viskosität der Lösung stark ansteigt. Nach 30 min. Rühren gibt man 5 ml ( 4,70 g / 37,2 mmol ) Allylmethacrylat hinzu und rührt weitere 30 min. Die Reaktion wird mit 2 ml Ethanol abgebrochen und die Trimethylsilylgruppen durch Rühren über Nacht mit 5 ml 10%iger wäßriger HCl-Lösung entfernt. Das Polymer wird ausgefällt, gewaschen und im Vakuum getrocknet.

Eine Molekulargewichtsbestimmung mittels GPC ergab ein Molekulargewicht von 30000 g/mol.

| **IR-Spektrum (cm**^{**-1**}**)** | | | |
|---|---|---|---|
| 3500-2500 | COOH | 3090 | =C-H |
| 3000-2900 | -C-H | 1740-1700 | C=O |
| 1650 | C=C | 980 + 920 | Allylgruppe |

| **1H-NMR Spektrum in DMSO-D6 (ppm)** | | | |
|---|---|---|---|
| 12,4 | bs | | -COOH |
| 5,9 | bs | 1H | |
| 5,3 | dd | 2H | Allylgruppe |
| 4,5 | bs | 2H | |
| 0,7-1,9 | m | | CH₂ + CH₃ (Polymerkette) |
| (dd doppeltes Duplett s Singulett bs broad Singulett m Multiplett) | | | |

Durch Auswertung der Integralverhältnisse ergibt sich ein Monomerverhältnis von ca. 1,9 (Methacrylsäuretrimethylsilylester) zu 1 (Allylmethacrylat) d.h. der Methacrylsäuretrimethylsilylester wird etwas schlechter eingebaut, vergleicht man das Einbauverhältnis mit dem Molverhältnis der Ausgangskomponenten (2,3:1). Löslichkeitsversuche in N-Methylpyrrolidon oder Ethanol zeigten, daß das Polymer nicht vernetzt ist.

Die Ergebnisse der Untersuchung lassen auf folgende Strukturformel schließen: 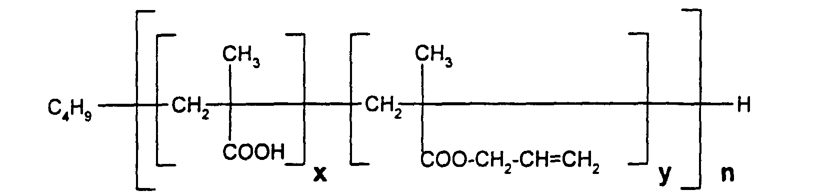 mit x = 180-200, y = 80-100; n = 1 Durch Vermischen der obigen pulverförmigen Substanz mit Ionomerglaspulver und etwas Wasser erhält man einen pastenförmigen Glasionomerzement, der innerhalb weniger Minuten zu einer gummiähnlichen Konsistenz und innerhalb einer halben Stunde zu einem harten Feststoff aushärtet.

## Patentansprüche

1. Polymerisierbare Oligo- und/oder Polyalkensäuren mit Blockstruktur, der allgemeinen Formel
**A-[-B**_{**x**}**-C**_{**y**}**-D**_{**z**}**-]**_{**n**}**-E ,**
wobei
a) die Gruppen **A** und **E** gleich oder verschieden Wasserstoff, Halogenatome, Methacrylat-, Acrylat-, Allyl-, Vinyl-, Alkyl-, Aryl-, Alkoxy-, Aryloxy-, Carboxyl-, Amin-, Hydroxy-, Isocyanat-, Silyl- und/oder Siloxygruppen sind,
b) die Gruppe **B** ein Segment bestehend aus Sequenzen von Mono-, Di- und/oder Tricarbonsäuren als Monomerbausteine, deren Anhydriden, Salzen und/oder Derivaten dieser Säuren mit einer an sich bekannten Säureschutzgruppe ist, aus denen die Säure leicht freizusetzen ist, und der Monomerbaustein für jedes n unterschiedlich sein kann,
c) die Gruppe **C** ein Segment bestehend aus Sequenzen von Mono-, Di- und/oder Tricarbonsäureestern und/oder deren Amiden als Monomerbausteine ist, wobei die Alkoholkomponente der Ester, sowie die Aminkomponente der Amide jeweils ungesättig ist und der Monomerbaustein für jedes n unterschiedlich sein kann,
d) die Gruppe **D** ein Segment ist bestehend aus Sequenzen von Mono-, Di- und/oder Tricarbonsäuren als Monomerbausteine, deren Estern, Amiden und/oder Nitrilen, die keine Doppelbindungen enthalten, und der Monomerbaustein für jedes n unterschiedlich sein kann,
e) die Abfolge der Gruppen B ,C und D innerhalb der Blöcke [-Bₓ-C_{y}-D_{z}-] beliebig ist und für jedes n unterschiedlich sein kann,
f) die Indizes n= 1 bis 10 und x, y und z = 0 oder 4 bis 1000 bedeuten, wobei für mindestens ein n x mindestens 4 und y mindestens 4 sind und x ,y und z für jedes n unterschiedlich sein können.
g) die polymerisierbaren Oligo- und/oder Polyalkensäuren mit Blockstruktur bzw. deren Vorläufer durch anionische Polymerisation herstellbar sind.

2. Polymerisierbare Oligo- und/oder Polyalkensäuren gemäß Anspruch 1, **dadurch gekennzeichnet,** daß die Gruppen B, C und D Segmente bestehend aus Sequenzen der Acrylsäure, der Methacrylsäure, der Maleinsäure, der Itaconsäure, sowie ggf. deren Ester, Anhydride, Amide, Nitrile und/oder Derivate sind, aus denen die Säuren freizusetzen sind.

3. Polymerisierbare Oligo- und/oder Polyalkensäuren gemäß einem der vorhergehenden Ansprüche, **dadurch gekennzeichnet,** daß die Säureschutzgruppen aus Anspruch 1b Trialkylsilylester und/oder -siloxyester sind.

4. Polymerisierbare Oligo- und/oder Polyalkensäuren gemäß einem der vorhergehenden Ansprüche, **dadurch gekennzeichnet**, daß die Alkoholkomponenten der Ester oder die Aminkomponenten der Amide aus Anspruch lc Vinyl, Allyl und/oder Vinyliden sind.

5. Polymerisierbare Oligo- und/oder Polyalkensäuren gemäß einem der vorhergehenden Ansprüche, **dadurch gekennzeichnet**, daß die Alkoholkomponenten der Ester oder die Aminkomponenten der Amide aus Anspruch ld Alkyl insbesondere Methyl, Ethyl, Propyl, tert.-Butyl und/oder substituiertes oder unsubstituiertes Aryl sind.

6. Polymerisierbare Oligo- und/oder Polyalkensäuren gemäß einem der vorhergehenden Ansprüche, **dadurch gekennzeichnet**, daß Gruppe C ein Segment bestehend aus oligomeren/polymeren Sequenzen des Methacrylsäureallyl- oder vinylester ist und die Gruppe B ein Segment ist bestehend aus oligomeren/polymeren Sequenzen des Methacrylsäuretrimethylsilylesters oder eines anderen Derivates dieser Säure, das leicht zur Säure hydrolisiert werden kann.

7. Polymerisierbare Oligo- und/oder Polyalkensäuren gemäß einem der vorhergehenden Ansprüche, **dadurch gekennzeichnet,** daß der Index n = 1 bis 4 bedeutet.

8. Anwendung einer oder mehrerer polymerisierbarer Oligo- und/oder polyalkensäuren gemäß einem der Ansprüche 1 bis 7 im dentalen und medizinischen Bereich.

9. Anwendung einer oder mehrerer polymerisierbarer Oligo- und/oder Polyalkensäuren gemäß einem der Ansprüche 1 bis 7 als Bestandteil von aushärtenden Formkörpern, Fissurenversiegelungs-Materialien, Haftvermittlern (Bonds) und oberflächenmodifizierenden Präparaten (Preps und Conditioner).

10. Anwendung einer oder mehrerer polymerisierbarer Oligo- und/oder Polyalkensäuren gemäß einem der Ansprüche 1 bis 7 als Komponenten in polymerisierbaren Glasionomerzementen.

## Claims

1. Polymerizable, block-structured oligo- and/or polyalkenoic acids of the general formula
**A-[-B**_{**x**}**-C**_{**y**}**-D**_{**z**}**-]**_{**n**}**-E**
wherein
a) groups **A** and **E**, being the same or different, are hydrogen, halogen atoms, methacrylate -, acrylate -, allyl -, vinyl -, alkyl -, aryl -, alkoxy -, aryloxy -, carboxyl -, amine -, hydroxy -, isocyanate -, silyl, and/or siloxy groups,
b) group **B** is a segment consisting of sequences of mono-, di-, and/or tricarboxylic acids as monomeric units, the anhydrides, salts, and/or derivatives thereof having an acid protective group known as such, from which the acid can be readily set free, and the monomeric unit can be different for each n,
c) group **C** is a segment consisting of sequences of mono-, di-, and/or tricarboxylic acid esters and/or the amides thereof as monomeric units, wherein the alcohol component of the esters and the amine component of the amides each are unsaturated and the monomeric unit can be different for each n,
d) group **D** is a segment consisting of sequences of mono-, di-, and/or tricarboxylic acids as monomeric units, their esters, amides, and/or nitriles not containing any double bonds, and the monomeric unit can be different for each n,
e) the order of groups B, C, and D within these blocks [-Bₓ-C_{y}-D_{z}-] is optional and can be different for each n,
f) the index **n** represents 1 to 10 and the indices **x**, **y**, and **z** are 0 or 4 to 1,000, wherein for at least one n x is at least 4 and y is at least 4 and x, y, and z can be different for each n, and
g) the polymerizable, block-structured oligo- and/or polyalkenoic acids and their precursors, respectively, producible by anionic polymerization.

2. Polymerizable oligo- and/or polyalkenoic acids according to claim 1 **characterized** in that the groups B, C, and D are segments consisting of sequences of acrylic acid, methacrylic acid, maleic acid, itaconic acid, and, respectively, their esters, anhydrides, amides, nitriles and/or derivatives from which the acids can be set free.

3. Polymerizable oligo- and/or polyalkenoic acids according to any one of the preceding claims **characterized** in that the acid protective groups defined in claim 1b are trialkylsilyl esters and/or -siloxy esters.

4. Polymerizable oligo- and/or polyalkenoic acids according to any one of the preceding claims **characterized** in that the alcohol components of the esters or the amine components of the amides defined in claim 1c are vinyl, allyl and/or vinylidene.

5. Polymerizable oligo- and/or polyalkenoic acids according to any one of the preceding claims **characterized** in that the alcohol components of the esters or the amine components of the amides defined in claim 1d are alkyl, particularly methyl, ethyl, propyl, tert-butyl, and/or substituted or unsubstituted aryl.

6. Polymerizable oligo- and/or polyalkenoic acids according to any one of the preceding claims **characterized** in that group **C** is a segment consisting of oligomeric/polymeric sequences of methacrylic acid allyl - or - vinyl ester and group **B** is a segment consisting of oligomeric/polymeric sequences of methacrylic acid trimethylsilyl ester or any other derivative of said acid which can be readily hydrolyzed to yield an acid.

7. Polymerizable oligo- and/or polyalkenoic acids according to any one of the preceding claims **characterized** in that the index n represents 1 to 4.

8. The use of one or more of the polymerizable oligo- and/or polyalkenoic acids according to any one of claims 1 to 7 for dental and medical purposes.

9. The use of one or more of the polymerizable oligo- and/or polyalkenoic acids according to any one of claims 1 to 7 as a constituent of curing molded articles, fissure sealants, adhesion promoters (bonds), and surface-modifying preparations (preps and conditioners).

10. The use of one or more of the polymerizable oligo- and/or polyalkenoic acids according to any one of claims 1 to 7 as components in polymerizable glass ionomer cements.

## Revendications

1. Acides oligo- et/ou polyalcénoïques polymérisables à structure séquencée, de formule générale :
A- [-Bₓ-C_{y}-D_{z}-]ₙ-E
dans laquelle
a) les groupes A et E, identiques ou différents, représentent de l'hydrogène, des atomes d'halogènes, des groupes méthacrylate, acrylate, allyle, vinyle, alkyle, aryle, alkoxy, aryloxy, carboxyle, amino, hydroxy, isocyanate, silyle et/ou siloxy,
b) le groupe B est un segment constitué de séquences d'acides mono-, di- et/ou tricarboxyliques comme motifs monomères, leurs anhydrides, leurs sels et/ou des dérivés de ces acides portant un groupe connu protégeant la fonction acide, desquels l'acide peut être libéré aisément, et le motif monomère peut être différent pour chaque n,
c) le groupe C est un segment constitué de séquences d'esters d'acides mono-, di- et/ou tricarboxyliques et/ou de leurs amides comme motifs monomères, le composant alcool des esters, de même que le composant amine des amides, étant insaturé dans chaque cas et le motif monomère pouvant être différent pour chaque n,
d) le groupe D est un segment constitué de séquences d'acides mono-, di- et/ou tricarboxyliques comme motifs monomères, de leurs esters, amides et/ou nitriles, qui ne contiennent pas de doubles liaisons, et le motif monomère pouvant être différent pour chaque n,
e) la succession des groupes B, C et D à l'intérieur des séquences [-Bₓ-C_{y}-D_{z}-] étant quelconque et pouvant être différente pour chaque n,
f) l'indice n ayant une valeur de 1 à 10 et les indices x, y et z ayant la valeur 0 ou une valeur de 4 à 1000, pour au moins un n, l'indice x est au moins égal à 4 et l'indice y est au moins égal à 4, et x, y et z peuvent être différents pour chaque n,
g) les acides oligo- et/ou polyalcénoïques polymérisables de structure séquencée ou leurs précurseurs peuvent être préparés par polymérisation anionique.

2. Acides oligo- et/ou polyalcénoïques polymérisables suivant la revendication 1, caractérisés en ce que les groupes B, C et D sont des segments constitués de séquences d'acide acrylique, d'acide méthacrylique, d'acide maléique, d'acide itaconique ainsi que, le cas échéant, de leurs esters, anhydrides, amides, nitriles et/ou dérivés, desquels les acides peuvent être libérés.

3. Acides oligo- et/ou polyalcénoïques polymérisables suivant l'une des revendications précédentes, caractérisés en ce que les groupes protégeant la fonction acide dans le b) de la revendication 1 sont des trialkylsilylesters et/ou des trialkylsiloxyesters.

4. Acides oligo- et/ou polyalcénoïques polymérisables suivant l'une des revendications précédentes, caractérisés en ce que les composants alcool des esters ou les composants amine des amides dans le c) de la revendication 1 sont les groupes vinyle, allyle et/ou vinylidène.

5. Acides oligo- et/ou polyalcénoïques polymérisables suivant l'une des revendications précédentes, caractérisés en ce que les composants alcool des esters ou les composants amine des amides dans le d) de la revendication 1 sont des groupes alkyle, en particulier méthyle, éthyle, propyle, tertiobutyle et/ou un groupe aryle substitué ou non substitué.

6. Acides oligo- et/ou polyalcénoïques polymérisables suivant l'une des revendications précédentes, caractérisés en ce que le groupe C est un segment constitué de séquences oligomères/polymères de l'ester d'allyle ou de vinyle de l'acide méthacrylique et le groupe B est un segment constitué de séquences oligomères/polymères de l'ester de triméthylsilyle de l'acide méthacrylique ou d'un autre dérivé de cet acide, qui peut être aisément hydrolysé en l'acide.

7. Acides oligo- et/ou polyalcénoïques polymérisables suivant l'une des revendications précédentes, caractérisés en ce que l'indice n a une valeur de 1 à 4.

8. Utilisation d'un ou plusieurs acides oligo- et/ou polyalcénoïques polymérisables suivant l'une des revendications 1 à 7 dans le domaine dentaire et médical.

9. Utilisation d'un ou plusieurs acides oligo- et/ou polyalcénoïques polymérisables suivant l'une des revendications 1 à 7, comme constituant de pièces moulées, de matériaux pour le bouchage de fissures, d'adhésifs (Bonds) et de préparations modificatrices de surfaces (Preps et Conditioner).

10. Utilisation d'un ou plusieurs acides oligo- et/ou polyalcénoïques polymérisables suivant l'une des revendications 1 à 7 comme composants de ciments ionomères vitreux polymérisables.
